# EUROPEAN PATENT APPLICATION

(11) **EP 1 496 463 A2**
(43) Date of publication of application: **12.01.2005**
(21) Application number: 04015114.4
(22) Date of filing: 28.06.2004
(51) Int. Cl.: G06F 19/00

(54) **Point of care information management system**

(30) Priority: 08.07.2003 US 614079
(71) Applicant: Becton, Dickinson and Company, Franklin Lakes, NJ 07417-1880 (US)
(72) Inventor: Swenson, Kirk D., North Caldwell, NJ 07006 (US); Stevens, Timothy A., Warwick, NY 10990 (US); Marsden, Stewart E., Montville, NJ 07045 (US); Crawford, Jamieson W.M., New York, NY 10025 (US)
(74) Representative: von Kreisler, Alek, Dipl.-Chem.

(57) **Abstract**

A system and method for collecting and testing data at a patient point of care location via a first device, such as a sample cartridge adapted to engage a first device, such as a personal digital assistant (PDA) incorporating a wireless communication module to communicate collected data to a second device, such as a healthcare workers personal PDA located beyond a contamination radius about a patient. Each device can further include a registration device, such as a bar code reader, to collect additional information at a point of care and tag data, or data analysis information, with an associated patient code.

## Description

### BACKROUND OF THE INVENTION

### 1. Field of the Invention

The present invention relates to a system for collecting and testing data at a patient point of care (POC) location via a first device, such as a sample cartridge, and using a first wireless communication module to communicate collected data to a second device located beyond a contamination radius about the patient. The second device can process the data and communicate results to a network. Each wireless communication module can also include a registration device, such as a bar code reader, to tag data or data analysis information, with an associated patient code.

### 2. Background of the Invention

In a typical healthcare environment, many testing apparatus can be used to perform patient tests for doctors and healthcare professionals. Such apparatus perform these tests, such as fluid sample tests, on patient fluids such as blood, urine, plasma, serum or other fluids, in order to measure chemical or physical properties of the samples and the results of these tests are used by doctors and healthcare professionals to make clinical decisions related to patient care and treatment. Because such results are used to make decisions for patient care, dependable test results are of the utmost importance. However, in addition to dependability considerations, many situations may require immediate determination of test results for effective care and treatment, resulting in more and more tests performed at the patient's bedside. Unfortunately, unlike a laboratory testing environment in which contamination risks may be controlled, patient point of care testing includes a substantial risk of contamination to healthcare workers, testing, data collection and data maintenance devices.

The Occupational Health and Safety Administration (OSHA) has published extensive rules regarding the protection healthcare workers from risks associated with occupational exposure to patient fluids in these situations. As noted by OSHA in 29 CFR, part 1910.1030, the entire content of which is incorporated herein by reference, a contaminated field includes the presence or the reasonably anticipated presence of blood or other potentially infectious materials on an item or surface. Typically, all human blood and certain human body fluids are treated as if known to be infectious for HIV, HBV, and other bloodbome pathogens. Therefore, at a typical point of care operation, a significant contamination field surrounds a patient. The rules promulgated by OSHA require precautions in association with such fields to prevent contact with blood or other potentially infectious materials located within such a contaminated field. Under circumstances in which differentiation between body fluid types is difficult or impossible, all body fluids shall be considered potentially infectious materials.

Such precautions typically ensure that the healthcare professional uses appropriate personal protective equipment unless, using professional judgment, its use would prevent the delivery of health care or public safety services or would have posed an increased safety hazard. Currently, the predominant means of protecting healthcare workers from contamination includes shielding devices, such as gloves, gowns and drapes. Gloves, such as latex and non-latex gloves, provide protection from contamination, however, are often misused. A healthcare worker can inadvertently keep the same pair of gloves on and transfer contaminants from one patient or device to another. Also, some patients and healthcare workers suffer an intolerance to latex, requiring the use of an alternate material, such as vinyl. A second device commonly used for protecting healthcare workers includes gowns, both single-use and multiple-use, that may have varying degrees of barrier protection depending upon the patient procedure. The American Society for Testing and Materials (ASTM) provides a standard test for gown resistance to synthetic blood and bloodborne pathogens. Additional information regarding such tests are discussed in ASTM Standard F1670 and Standard F1671, the entire content of each being incorporated herein by reference. A third device commonly used for protecting healthcare workers and establishing a sterile field about the patient includes drapes. Typical drape materials include layered cotton and plastic, sufficient to resist tearing and provide a sterile field for the table and instrument stands.

Instruments, such as sample collection devices used for collection, handling and testing of patient samples at a patient point of care, are also often subject to contamination during use. Many such devices however, include sensitive and costly electronics which make safe reuse difficult but required. Such devices are not designed to be widely disposable, or easily decontaminated. One such instrument currently available is a hand-held analyzer, which may be configured to accept samples contained within a standard collection device. Hand-held analyzers for sample testing at a patient point of care are extensively discussed in U.S. Patent No. 6,066,243 issued to Anderson et al., and in U.S. Patent Application Publication No. U.S. 2002/0002326 issued to Causey et al., the entire contents of each being incorporated herein by reference. Analyzers such as PDA-based devices are very cost effective, easily upgraded and allow on the spot analysis, but are also subject to the field contamination described above. Additional details regarding such PDA use at a patient point of care are discussed in U.S. Patent No. 5,096,669 issued to Lauks et al., in an article by Jason Thibeault entitled "Move Toward PDA-Based Devices Gets Boost from FDA", Medical Device & Diagnostic Industry, August 2002, in an article by Ian Austin entitled "Palmtops In The Operating Room", New York Times, August 22, 2002, and in an article by Stephanie De Ritis entitled "Expanding Exceeding POCT Boundaries", Advance/Laboratory, August 2002, the entire content of each being incorporated herein by reference.

The use of such devices within a contamination field create a unique problem when OSHA rules are applied. As noted in 29 CFR part 1910.1030(d)(4)(ii), all equipment and environmental and working surfaces are to be cleaned and decontaminated after contact with blood or other potentially infectious materials, possibly including any analytical device, hand-held personal digital assistant, or stand-alone computer workstation used at the patient point of care. Equipment which may become contaminated with blood or other potentially infectious materials is to be examined prior to servicing or shipping and must be decontaminated as necessary. Currently, a very narrow exception to the above rule exists, as noted in part 1910.1030(d)(2)(xiv), where decontamination of such equipment or portions of such equipment is shown to be not feasible. However, this exception applies to servicing or shipping contaminated equipment, therefore a need exists to minimize the contamination of advanced technology testing devices at patient point of care use to satisfy OSHA regulation 1910.1030(d)(4)(ii).

Accordingly, a need exists to provide a system and method for directly collecting and testing fluid samples such as blood, and communicating sample data without exposing the hand-held analytical device or stand-alone computer workstation to the contaminants at the patient point of care. The system and method should also provide a data management system for the communicated sample data, and include additional patient data provided by a patient identifier, such as a bar code.

### SUMMARY OF INVENTION

An object of the present invention is to provide a system and method for directly collecting and testing fluid samples such as blood, and communicating sample data without exposing the hand-held analytical device or stand-alone computer workstation to the contaminants at the patient point of care.

Another object of the present invention is to provide a system and method for a data management system for the communicated sample data, and include additional patient data provided by a patient identifier, such as a bar code

Another object of the present invention is to provide a system and method for a sample cartridge and wireless communication module which, when engaged, can test samples within a contamination field at a patient point of care, and communicate the results to a remote hand-held analytical device or stand-alone computer workstation beyond the contamination field.

Another object of the present invention is to provide a system and method for a patient bar code identification and wireless communication module which, when engaged, can assign patient information to test samples and results.

Another object of the present invention is to provide a system and method for a sample cartridge assembly that includes an identification element to inform a receiving module as to the cartridge type.

Another object of the present invention is to provide a system and method for a sample cartridge assembly that includes an identification element to inform a receiving module as to the tests to perform upon the cartridge.

These and other objects are substantially achieved by providing a system and method for receiving sample data at a central device adapted to maintain at least one database. The sample data is provided by at least one sample testing device at a patient point of care location, which is adapted to engage a sample cartridge and provide said sample data. The data is communicated from within a contamination field about a patient to the central device using wireless communication modules. The communication can be modulated, which allows the central device to communicate with multiple sample testing devices simultaneously. Cartridge identifier information and patient identifier label information is also provided to ensure the data maintained at the central device is accurate.

### BRIEF DESCRIPTIONOF THE DRAWINGS

These and other objects, advantages and novel features of the invention will be more readily appreciated from the following detailed description when read in conjunction with the accompanying drawings, in which:

Fig. 1 is a view of a contamination field about a patient point of care illustrating an implementation of a data collection and communication network according to an embodiment of the present invention;

Fig. 2 is a view of a contamination field about a patient point of care illustrating another example of an implementation of a data collection and communication network according to an embodiment of the present invention;

Fig. 3 is a view of a contamination field about a patient point of care illustrating yet another example of an implementation of a data collection and communication network according to an embodiment of the present invention;

Fig. 4 is a view of multiple contamination fields about one or more patient points of care illustrating still another example of an implementation of a data collection and communication network according to an embodiment of the present invention; and

Fig. 5 is a view of multiple contamination fields about one or more patient points of care illustrating still another example of an implementation of a data collection and communication network according to an embodiment of the present invention.

In the drawing figures, it will be understood that like numerals refer to like structures.

### DETAILED DESCRIPTION OF THE PREFERRED EMBODIMENTS

The embodiment of the present invention described below includes an array of communication modules which engage testing components and samples that previously would require contact with one another to function. Such required contact typically resulted in the use of many devices, such as a personal digital assistant (PDA) within a contamination radius about a patient point of care, which unfortunately can lead to contaminated devices. However, in the present invention, by creating a non-contact sample testing environment having wireless data transfer capabilities, the present invention allows a significant amount of the test equipment to remain beyond the contamination field about a patient. This helps healthcare professionals collect and review data at a patient point of care without the risk of contamination to themselves, or their personal data equipment. Additionally, as test data is communicated via wireless communication, data collection and management abilities are greatly improved.

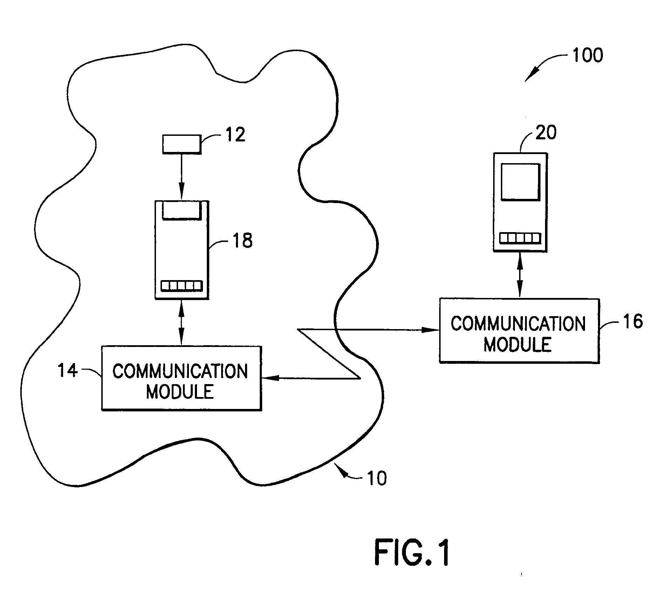

A diagram of an exemplary communication network 100 in accordance with an embodiment of the present invention is shown in Fig. 1. In Fig. 1, a sample cartridge 12 is located within a potential contamination field 10 about a patient. As noted earlier, the contamination field 10 can include the presence, or the reasonably anticipated presence, of blood or other potentially infectious materials on any item or surface. The field 10 can include contaminated laundry, which has been soiled with blood or other potentially infectious materials, and contaminated sharps, such as needles and scalpels that can easily penetrate the skin or latex gloves of healthcare professionals. Additionally, any sample collection cartridge or collection device associated with the patient, and any analytical device used with the cartridge within the field 10, would also typically become contaminated during use.

The contamination field 10 typically results from any number of procedures executed at a patient point of care, including fluid collection as part of a test procedure. In Fig. 1, a sample cartridge 12 is provided as part of such a patient point of care test procedure. For example, the sample cartridge 12 can be used to collect a fluid sample from the patient for testing using a hand-held analytical device or a stand-alone computer workstation. Additional details of such sample collection and testing using sample cartridges are discussed in a U.S. Patent Application of Stevens et al., entitled "Collection Device Adapted To Accept Cartridge For Point Of Care System", Attorney Docket No. P-5689/1 (43698), and in U.S. Patent Application of Stevens et al., entitled "Collection Device Adapted To Accept Cartridge For Point Of Care System", Attorney Docket No. P-5689/2 (43699), the entire content of each being incorporated herein by reference.

In Fig. 1, the sample cartridge 12 can be filled with a collected sample from a patient and thereafter, provided to perform a series of tests. The sample cartridge 12 provides a containment chamber into which fluid samples are placed, either directly or by using a collection device, for analysis. Once a sample is collected into the cartridge 12, the cartridge can be engaged with a remote analytical device 18 for testing and communicating test results and/or additional sample information to similar devices located beyond the contamination field 10. In the example of Fig. 1, the sample cartridge 12 provides contact between collected fluid samples and sensory apparatus, such as miniaturized electrodes and micro-sensors, for executing a series of diagnostic tests on the sample. The cartridge 12 can also include biosensors for performing electrochemical measurements, such as potential, current and conductivity measurements on the collected fluid sample.

When the sample cartridge 12 is prepared for testing, the sensory apparatus of the cartridge can be electrically coupled with a remote analytical device 18, such as a hand-held analyzer, personal digital assistant (PDA) or VISOR®. Alternatively, the cartridge 12 may be coupled with a stand-alone computer workstation, or with any number of existing analyzers, such as those manufactured by the I-Stat Corporation of Princeton New Jersey. In the embodiment shown in Fig. 1, the cartridge 12 is coupled with a PDA, or remote analytical device 18, which is however, subject to contamination by either being present within the contamination field 10, by contacting the sample cartridge 12, or by contacting a contaminated glove of a healthcare professional during testing. Therefore, the remote analytical device 18 can be uniquely constructed to be readily cleaned and decontaminated for multiple uses, and the sample cartridge 12 can be simply discarded after use.

Sample testing in accordance with the present invention is achieved by engaging the filled sample cartridge 12 with the remote analytical device 18, and once coupled, activating the remote analytical device to gather and process information regarding the contained sample via an array of sensory apparatus contained within the sample cartridge body. Where testing is to be completed within the contamination field 10, the remote analytical device 18 can produce outputs based upon test results which are displayed on an output mechanism, such as a liquid crystal display (LCD), analog display or light emitting diode (LED) indicator. Additional tests on the sample may be directed by activation of user interface mechanisms located on the analytical device. As noted above, the cartridge 12 is inexpensive and entirely disposable, and upon completion of sample testing, is removed from the analytical device and discarded. The remote analytical device 18 however, is subjected to a cleaning procedure as required by OSHA regulations referenced above.

Commonly, the healthcare worker may wish to use their personal PDA to perform sample tests or collect test information as described above. However, this could create a situation in which the personal PDA becomes contaminated, either through use within the contaminated field, or through contact with the patient, or patient fluids. In this case, the risk of spreading a bloodbome pathogen such as hepatitis B virus (HBV) and human immunodeficiency virus (HIV) is greatly increased. Also, the ability to clean such devices as required by OSHA can be limited, as such personal PDA devices are not typically designed for decontamination.

To allow healthcare workers to use a personal PDA device in sample testing without the risk of contamination, the embodiment of the present invention shown in Fig. 1 includes a wireless communication module 14 which is incorporated with the remote analytical device 18 and sample cartridge 12. The wireless communication module 14 includes a communication mechanism, such as set forth in the IEEE Standard 802.11 for wireless communications, the entire content of which is incorporated herein by reference, to receive instructions and broadcast sample data and test results generated by the remote analytical device 18 to at least a second communication module 16, as described in greater detail below. The wireless communication between modules 14 and 16 of Fig. 1, allows the sample cartridge 12 and remote analytical device 18 to remain within the contaminated field 10 about the patient during testing, and minimizes the contamination of additional devices, such as the healthcare workers personal PDA device receiving test results and information.

Such wireless communication has become increasingly prevalent over the past decade, and typically includes one or more transceivers that are capable of transmitting and receiving electromagnetic signals, such as radio frequency (RF) communications signals, to and from other transceivers located within a coverage area. The communication signals between transceivers can include, for example, any data that has been modulated according to a desired modulation technique and transmitted as data packets. The transceivers, which include at least one transmitter and receiver, can transmit and receive such data packet communications in any suitable format, such as a multiplexed format including time-division multiple access (TDMA), code-division multiple access (CDMA), or frequency-division multiple access (FDMA), each of which enables a single transceiver to communicate simultaneously with several other transceivers within a coverage area. In Fig. 1, communication modules 14 and 16 each include a transceiver which is capable of receiving and transmitting signals, such as the various formatted data signals described above, to and from the communication module. The data signals can include data or multimedia information and packetized control and data information. Each communication module 14 and 16 can also include the appropriate hardware and software to perform various communication functions, such as Internet Protocol (IP), transmission control protocol (TCP), user datagram protocol (UDP) and Address Resolution Protocol (ARP), the purposes of which can be readily appreciated by one skilled in the art. The communication modules are then incorporated with a host device 18 and 20, which can consist of any number of devices, such as a hand-held analytical device, personal digital assistant (PDA), stand-alone computer workstation, or any other suitable device to direct the testing and information collection from the sample cartridge 12, and then provide data for communication.

Returning to Fig. 1, the communication module 14, as noted above, is incorporated with a PDA or PDA-like remote analytical device 18, which can engage the sample cartridge 12 and direct the testing of the sample provided. The communication module 16, being beyond the contamination field 10, is incorporated with a second device, such as a hand-held analyzer, stand-alone computer workstation, or in the preferred embodiment, a healthcare worker's PDA 20. These devices are provided as examples only however, and can include any number of suitable devices for data collection and evaluation as required in the specific application.

Where few or no tests are to be performed and mere data communication from within the contamination field 10 is the primary goal, the PDA 18 can be eliminated, and the communication module 14 can be coupled with the sample cartridge 12 via a simple adapter (not shown), which directs data collection from the sample cartridge and provides the data for transfer to the module 14 and thereafter, communication to module 16 beyond the field 10. To facilitate this, each communication module 14 and 16 is compatible with a large number of such sample containing devices, allowing a broader point of care use of existing devices.

Unless specifically constructed to receive a sample cartridge, the compatibility of the remote analytical device 18 with testing apparatus, such as the sample cartridge 12, typically requires an interface mechanism. As known to those skilled in the art, many interface modules are provided to adapt hand-held devices to multiple uses, such as SPRINGBOARD® expansion modules for a PDA or VISOR®. Where required, such an adapter can be installed on the remote analytical device 18 to allow engagement with the sample cartridge 12. As noted for the communication module above, such an adapter allows the use of a wider range of collection cartridges with the remote analytical device 18. For example, this allows collection cartridges by manufacturers such as the I-Stat Corporation, to be used in place of the collection cartridge 12 described above.

Once engaged, the analytical device 18 includes hardware and software adapted to access an array of sensory apparatus within the cartridge 12 and gather information on the collected patient sample contained therein, such as pH, pCO₂, pO₂ Na⁺, Ca⁺⁺, K⁺, hematocrit and glucose levels in the sample, in addition to sample temperature measurements. Testing of the sample can be directed using the remote analytical device 18, or directed by the PDA 20 from beyond the contamination field 10 as described below. Additional details of directing a hand-held analytical device during sample testing are discussed in the U.S. Patent Applications of Stevens et al., referenced above.

Upon completion of the sample evaluation, the results and sample information can be communicated from the remote analytical device 18 to the communication module 14 as a stream of data. The data stream can then be prepared for transmission, including modulation according to a desired modulation technique, and thereafter transmitted as data packets by module 14 to module 16. As noted above, the use of any one of several available modulation techniques allows a single communication module to communicate simultaneously with several modules within a coverage area. Therefore, the PDA 20, or in the case of a broader application, a stand-alone computer workstation, is not limited to communications with a single sample testing communication module, such as shown in Fig. 1. Through signal modulation, the PDA 20 can communicate simultaneously with multiple modules, allowing greater data collection and management.

Each communication, or transmission of sample data between communication modules 14 and 16 is initiated as a data stream provided by the remote analytical device 18 to the communication module 14. As known to those skilled in the art, a communication module 14 can construct one or more information packets corresponding to a packet of data, wherein typically every packet is constructed with the same predetermined length (e.g., 128 bytes). When transmitting a data message, the first packet is constructed with a header section formed in a long header structure, followed by a data field. The long header typically includes a short command field, followed by a user identifying field, and a message total field. The command field identifies the process to be performed upon the subsequent data, the user ID identifies the communication module transmitting or receiving the packet, and the message total identifies the total length of the message which will follow. This total length includes all bytes within subsequent packets corresponding to this specific message, followed by a data field. If the message includes more data than will fit in a single packet, subsequent packets are transmitted.

Returning to Fig. 1, the test results and sample data are received at the second communication module 16 and provided to the hand-held analyzer, stand-alone computer workstation, or PDA 20. The communication path established between modules 14 and 16 also allows the PDA 20 to provide instructions and direct additional tests to be performed at the remote analytical device 18. In a similar fashion, the remote analytical device 18 can provide sample information for more extensive analysis at the PDA 20, rather than at the remote analytical device 18, which may lack a sufficient level of hardware or software to perform a full range of tests on the sample cartridge 12. Upon completion of initial tests and any additional tests directed by the PDA 20, the cartridge 12 and sample therein, are removed from the remote analytical device 18 and discarded. The analytical device 18 can then be cleaned and decontaminated for another use.

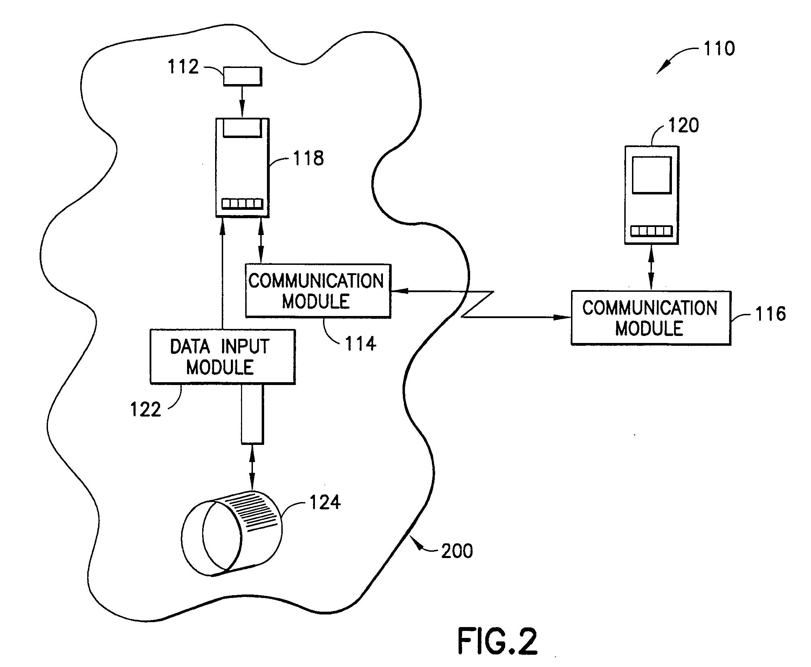

In a second embodiment of the present invention shown in Fig. 2, a data input device 122, such as a bar code reader and software, is provided to allow additional patient data to be included in the analysis performed by the remote analytical device 118. As shown in Fig. 2, a communication network 110 includes a sample cartridge 112 which is located within a potential contamination field 200 about a patient, and as noted above, the contamination field 200 can include the presence of blood or other potentially infectious materials on any item or surface. The sample cartridge 112 is provided to collect a fluid sample from the patient for testing, using the remote analytical device 118 substantially the same as described above in Fig. 1.

In Fig. 2, the second embodiment of the present invention includes a first wireless communication module 114 which is used to engage the remote analytical device 118 and includes a communication mechanism as described above, to broadcast test results and sample data to a second communication module 116. As in Fig. 1, the wireless communication between modules 114 and 116 allows the sample cartridge 112 to remain within the contamination field about the patient, and minimizes the contamination of additional testing devices and the risk to healthcare professionals. The patient of Fig. 2 (not shown) however, is also provided with an identifier 124, such as a bar code or a radio frequency identification (RFID) label, typically provided for patient data management purposes. The bar code or RFID label can be used to easily manage a large amount of patient information, which can be readily accessed at the patient point of care. As known to those skilled in the art, bar codes can be used to provide identification data in a number of formats, and are often provided to patients to assist in data management. For example, a Palm OS software application provided by BD (formerly Becton, Dickinson and Company) uses a barcode scanner to collect medication and patient barcode information for timely access by healthcare workers via handheld devices. Such information provided by a patient bar code can also include the name, age, medical background and special instructions regarding the patient. Such information as previous sample test intervals and test results are also valuable information that can be included on a patient bar code identifier 124. Such information can be of great importance in evaluating test results, as a single static value may not indicate a condition as clearly as a series of test results. The use of bar code scanning software incorporated with hand-held devices is further discussed in a paper by Pamela Van Hook entitled "Palm OS: Trends, Devices, Software, and Industry Applications", 2002, the entire content of which is incorporated herein by reference.

For these, and other reasons, the second embodiment of the present invention shown in Fig. 2 includes the additional data input device 122 which can be incorporated with the analytical devices of Fig. 2 in a number of ways as described in greater detail below.

As shown in Fig. 2, the data input device 122 is incorporated with the remote analytical device 118, and can be located within the contamination field 200 and therefore subject to the decontamination difficulties described above. However, in this position, the data input device 122 can be adjacent to the patient at the point of care, and can allow easier scanning of the patient bar code identifier 124 which is typically located on or very near the patient.

Scanning the identifier 124 provides the additional patient information for use by the remote analytical device 118 primarily during engagement with the sample cartridge 112. This allows the remote analytical device to perform tests, including the additional information. Test results and sample information is "tagged" with a patient identifier, such as a name or identifying number prior to communication. When the test results and sample information are then communicated to the second communication module 116 and the associated PDA 120, the patient identifier information is included. Although the additional information provided by scanning the patient identifier 124 is primarily used in association with the sample testing, the second embodiment of the present invention allows the scanning and communication of identifier information at any time, regardless of testing situations.

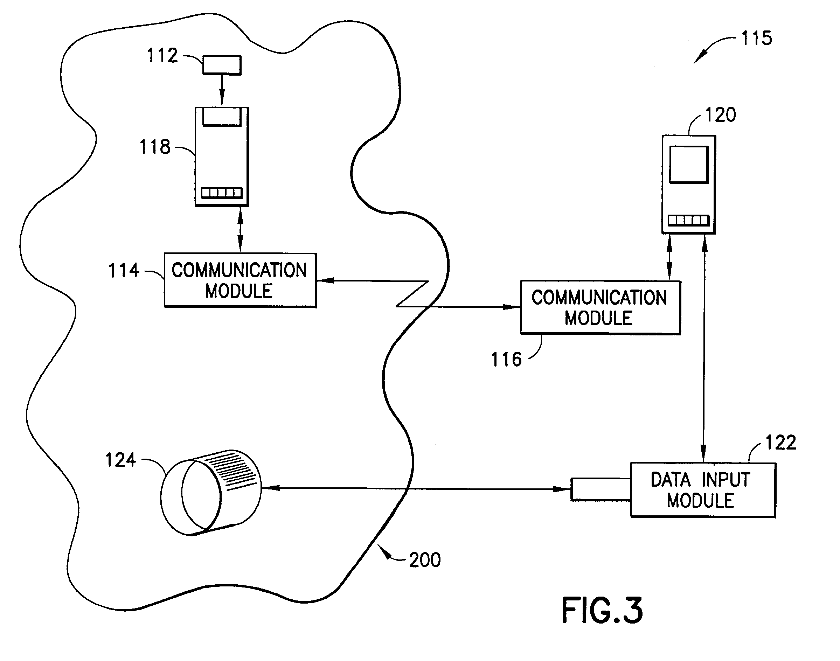

In Fig. 3, a communication network 115 illustrates a third embodiment of the present invention wherein the data input device 122 is incorporated with the PDA 120. In this configuration, the data input device 122 is typically not located within the contamination field 200, or is used briefly within the contamination field, therefore is not subject to the decontamination difficulties described above. Scanning the identifier 124 provides the additional patient information to the PDA 120 therefore, after the remote analytical device 118 communicates test results or sample information to the PDA 120, the information is tagged at the PDA 120 with the patient identifier information.

Still another identifier technique which can be used in the second and third embodiments of the present invention, include RFID labels (not shown), which can be used as an identifier 124 to provide similar information. An RFID label allows the healthcare professional to edit, or "write" new data to the label after each test. An RFID label is a film-label in which a microchip and an antenna are integrated. All data is saved on the memory microchip, which can be automatically read and modified by a contact free read-write device. In each embodiment of the present invention described above, the transceiver of communication modules 114 and 116 can operate as a reader, or "interrogator", and decode the data encoded in the label's integrated circuit and pass the data to the PDA 120 for processing and tagging as described above. With such a technique, the healthcare professional is not required to handle the label 124 or enter the contamination field. Either communication module 114 or 116 can read the data contained on the label 124 and provide the data to the PDA 120 for data management purposes. After testing of the sample cartridge 112 is complete, the PDA 120 can direct the update, or "writing" of the label 124, via the communication module 116 with the newly produced test information, thereby ensuring label data is current, while protecting healthcare workers and equipment from contamination risks. If the label 124 is beyond a range which allows module 116 to write information to the label, the PDA 120 can direct the module 114 to write the new data to the label.

Additional information which can be generated through the sample cartridge evaluation described above also includes a data log of sample cartridge engagements. Each sample cartridge can include an identifier to notify the analytical device of cartridge type and function being used. This information can also be used to determine which tests, and sample data, is to be collected from the sample cartridge, as specialty cartridges can be manufactured to locate and identify only particular sample information. In these cases, a full test may produce abnormal results, as the cartridge is not adapted to provide full results. These abnormal results, if communicated by the remote analytical device, could adversely affect data identified with that patient.

Such sample cartridge identifiers can be constructed using the existing electronics located within the cartridge or using other suitable techniques. For example, one form of cartridge identifier can include the shorted connection or open circuit connection of one or more pairs of sensor conductors within the cartridge that are engaged by the analytical device during testing. Such pairs, known by the analytical device to indicate a particular sample cartridge identity, can be monitored prior to testing. Based upon the detection of the cartridge identity, the analytical device can direct a specific sample test or data collection. When the results and data are transmitted, additional information regarding the cartridge, such as cartridge type and manufacture, can be included.

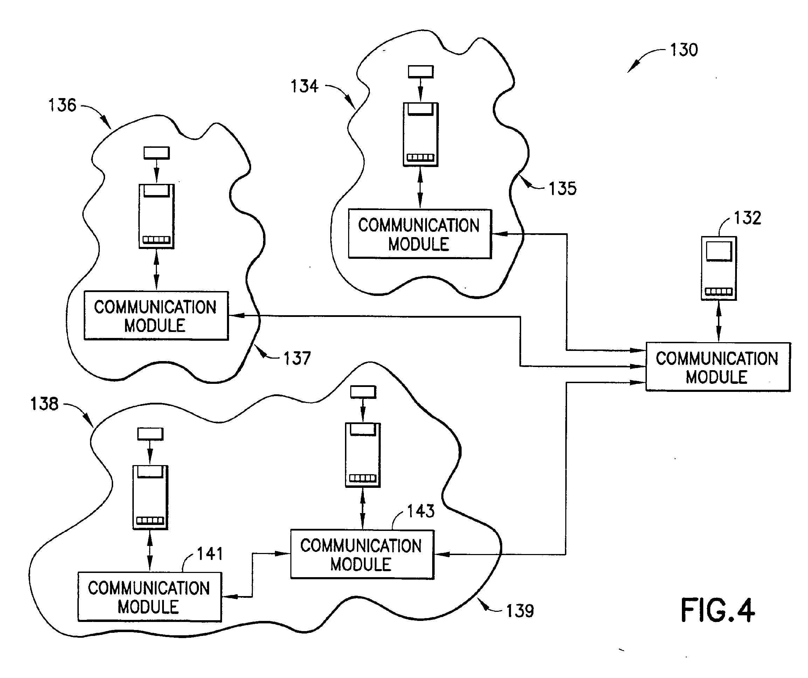

As noted above, through signal modulation, the PDA device beyond the contamination field of Figs. 1, 2 and 3 can communicate simultaneously with multiple modules, allowing greater data collection and management. As shown in Fig. 4, a communication network 130 includes a single PDA 132 which can simultaneously collect test results and sample data from numerous remote analytical devices and sample cartridges 134 through 138. Each communication can also be tagged with patient identifier information provide by a patient identifier as described above. Where possible, the PDA 132 can also update patient identifiers either directly or via one or more distributed communication modules. Such updates can take place when new data becomes available or at any periodic interval. As a precaution, the cartridge identifier serves to minimize the risk of the introduction of inaccurate information to the PDA 132.

In the embodiments described above, the PDA 132 can request information from each remote analytical device, however, communication can be configured to occur automatically whenever communication modules detect another within communication range. Therefore, the PDA 132 user need not specifically request information as updated information is communicated any time the PDA 132 is within communication range of a remote analytical device. In a similar fashion, the data accumulated by the PDA 132, or any remote analytical device, can be automatically transferred via wireless communication to a central computer workstation as described below.

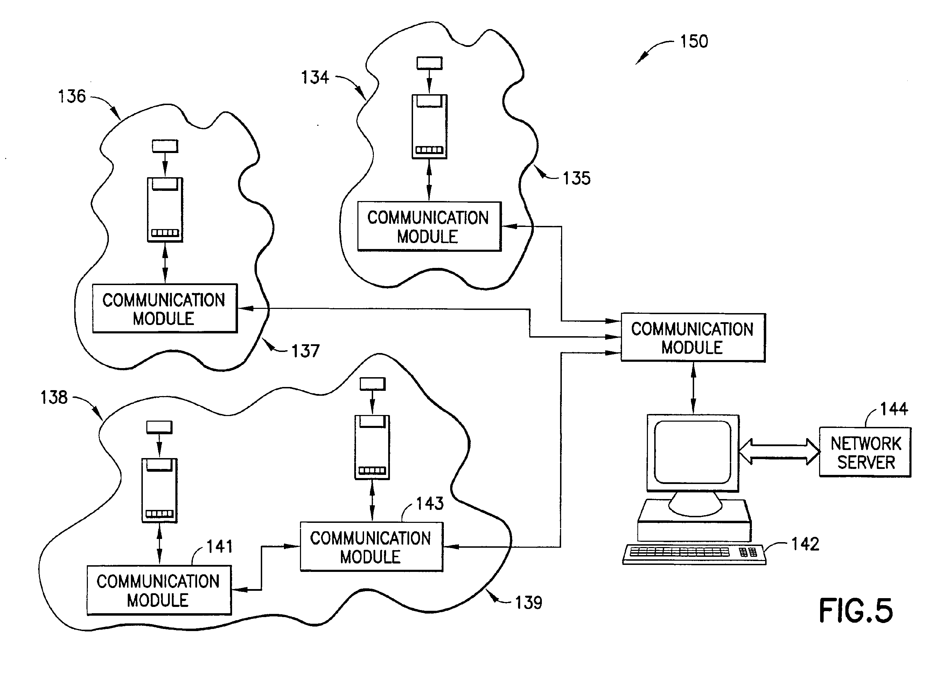

As shown in Fig. 5, a communication network 135 includes a central computer workstation 142 which can be used to simultaneously collect test results and sample data from the numerous remote analytical devices and sample cartridges 134 through 138, as described above in Fig. 4. The resulting collected information can be managed in a variety of ways and provided to healthcare workers in a dynamic, continuously updated fashion. Through the central workstation 142, the communication modules can access a fixed network or server and communicate with other networks, such as the Internet. In such an implementation, test results at a patient point of care can be provided to virtually any location. As shown in Fig. 5, the central computer workstation 142 can serve as the interface between each point of care and the network server 144 in the communication network 135, and can further provide updated information to each remote analytical device or PDA within communication range. This continuous update, or "downloading of information" to accessible PDA devices ensures healthcare workers moving about the communication network are continuously receiving information, regardless of requests or participation within a contamination field at multiple patient point of care locations.

The automatic communication of data can be further improved by incorporating yet another communication technique at each remote analytical device in the embodiments described above. The communication modules can be implemented using wireless local area networks (WLANs) and, in particular, 802.11 wireless LANs, which also include a peer-to-peer mode that enables modules to communicate directly with one another. This allows each remote analytical device to receive and route data from other communication modules, through a technique commonly referred to as "hopping". For example, as shown in Figs. 4 and 5, should the communication module 141 be located beyond a direct communication range of the computer 142 or PDA 132, data can still be received by routing data from module 141 through module 143, and to the computer 142 or PDA 132.

Although only a few exemplary embodiments of the present invention have been described in detail above, those skilled in the art will readily appreciate that many modifications are possible in the exemplary embodiments without materially departing from the novel teachings and advantages of this invention. Accordingly, all such modifications are intended to be included within the scope of this invention as defined in the following claims.

## Claims

1. A method of collecting and testing data from a plurality of patient point of care locations, the method comprising:
controlling a central device to receive sample data from at least one sample testing device at a patient point of care location, said sample testing device adapted to engage a sample cartridge and provide said sample data, said central device adapted to maintain at least one database;
controlling said central device to receive cartridge identifier information from said sample testing device;
controlling said central device to tag said received sample data with a patient identifier label information, said patient identifier label information communicated to said central device via a data input device; and
controlling said central device to update said database based upon at least one of said received sample data, cartridge identifier information and patient identifier information, and provide said database to a network server.

2. A method of collecting and testing data from a plurality of patient point of care locations as claimed in claim 1, further comprising:
controlling said sample testing device to communicate said sample data to said central device as at least one data packet communicated from said sample testing device via a first wireless communication module.

3. A method of collecting and testing data from a plurality of patient point of care locations as claimed in claim 1, further comprising:
controlling said central device to receive said sample data from a plurality of sample testing devices simultaneously via a second wireless communication module, and controlling said central device to communicate data to said sample testing device as at least one data packet communicated from said central device via said second wireless communication module.

4. A method of collecting and testing data from a plurality of patient point of care locations as claimed in claim 1, further comprising
controlling said central device to communicate data to said patient identifier information label as at least one data packet communicated from said central device via a second wireless communication module.

5. A method of collecting and testing data from a plurality of patient point of care locations as claimed in claim 6, wherein said patient identifier information label is a radio frequency identification label, and wherein said data input device is at least one of a bar code reader and a radio frequency identification label.

6. A method of collecting and testing data from a plurality of patient point of care locations as claimed in claim 1, wherein said data input device is incorporated with said sample testing device.

7. A method of collecting and testing data from a plurality of patient point of care locations as claimed in claim 1, wherein said data input device is incorporated with said central device.

8. A method of collecting and testing data from a plurality of patient point of care locations as claimed in claim 1, wherein said testing device comprises at least one of a hand-held analytical device and stand-alone computer workstation, said testing device located within a contamination field about a patient at a patient point of care location.

9. A method of collecting and testing data from a plurality of patient point of care locations as claimed in claim 1, wherein said central device comprises at least one of a hand-held analytical device and stand-alone computer workstation, said central device located beyond a contamination field about a patient at a patient point of care location.

10. A system, adapted to collect and test data at a patient point of care location from a point located beyond a contamination radius about a patient using modular components to create a point of care network, the system comprising:
a sample cartridge, adapted to engage a sample testing device for testing a collected sample at a patient point of care location, said sample cartridge including a cartridge identifier mechanism, adapted to provide cartridge identifier information;
a patient identifier label, adapted to provide patient identifier information; and
a central device, adapted to receive sample data from said sample testing device at a patient point of care location, said central device being further adapted to maintain at least one database and to update said database based upon at least one of said cartridge identifier information, patient identifier information, and received sample data, and to provide said database to a network server.

11. A system as claimed in claim 10, wherein:
said central device is further adapted to tag said received sample data with said patient identifier label information.

12. A system as claimed in claim 10, wherein:
said sample testing device is adapted to communicate said sample data to said central device as at least one data packet communicated from said sample testing device via a first wireless communication module.

13. A system as claimed in claim 10, wherein:
said central device is adapted to receive said sample data from a plurality of sample testing devices simultaneously via a second wireless communication module, and said central device is adapted to communicate data to said sample testing device as at least one data packet communicated from said central device via said second wireless communication module.

14. A system as claimed in claim 10, wherein
said central device is adapted to communicate data to said patient identifier information label as at least one data packet communicated from said central device via a second wireless communication module.

15. A system as claimed in claim 10, and wherein said data input device is at least one of a bar code reader and a radio frequency identification label.

16. A system as claimed in claim 10, wherein said data input device is incorporated with said sample testing device.

17. A system as claimed in claim 10, wherein said data input device is incorporated with said central device.

18. A system as claimed in claim 10, wherein said testing device comprises at least one of a hand-held analytical device and stand-alone computer workstation, said testing device located within a contamination field about a patient at a patient point of care location.

19. A system as claimed in claim 10, wherein said central device comprises at least one of a hand-held analytical device and stand-alone computer workstation, said central device located beyond a contamination field about a patient at a patient point of care location.
